# DEMANDE DE BREVET EUROPEEN

(11) **EP 1 005 828 A1**
(43) Date de publication de la demande: **07.06.2000**
(21) Numéro de dépôt: 99403008.8
(22) Date de dépôt: 02.12.1999
(51) Int. Cl.: A61B 5/044

(54) **Procédé et dispositif d'enregistrement synchrone d'électrocardiogrammes et d'images vidéo**

(30) Priorité: 02.12.1998 FR 9815231
(71) Demandeur: IODP S.a.r.l., 75005 Paris (FR)
(72) Inventeur: Massonneau, Marc, 75005 Paris (FR)
(74) Mandataire: Breese, Pierre

(57) **Abrégé**

L'invention concerne un procédé de traitement de données biomédicales constituées par au moins un signal vidéo provenant d'un système d'imagerie médical synchrone avec au moins un signal bioélectrique acquis par au moins un capteur bioélectrique caractérisé en ce que le signal bioélectrique constitue le signal de modulation d'un signal audiofréquence et en ce que le signal ainsi modulé est transmis de manière synchrone avec le signal vidéo.

## Description

La présente invention concerne un procédé pour le traitement combiné de données biomédicales différentes, notamment les données contenant des images vidéo comme l'échographie, et les données se traduisant par un signal électrique, comme l'électrocardiogramme.

On connaît dans l'art antérieur des moyens de mesurer, d'enregistrer et de transmettre indépendamment les données biomédicales d'un patient. Cependant, le praticien doit effectuer lui-même les comparaisons entre ces différents informations à un temps donné et ne dispose pas de moyens permettant une observation synchronisée des éléments dont il dispose. Il ne peut ainsi pas observer les corrélations qui pourraient exister et qui pourraient lui permettre d'affiner son diagnostic.

Une solution est enseignée dans la demande de brevet européen EP86400739.8, laquelle concerne un dispositif d'observation et d'enregistrement de l'activité d'au moins un organe du corps d'un patient se traduisant par des manifestations électriquement enregistrables, en vue notamment de l'étude et de la surveillance de maladies cardiaques, comportant des moyens vidéo de prise de vue des mouvements du patient, des moyens de détection et de transmission de signaux électriques traduisant l'activité de l'organe considéré, des moyens de combinaison des signaux vidéo correspondant à l'image du patient et des signaux électriques traduisant l'activité dudit organe, des moyens d'enregistrement desdits signaux combinés et des moyens d'affichage simultané des images relatives aux mouvements du patient et de celles des signaux électriques traduisant l'activité dudit organe. Ce dispositif est caractérisé en ce que les moyens de détection et de transmission des signaux électriques traduisant l'activité dudit organe comprennent, comme connu en soi, des capteurs fixés au corps du patient de façon à recevoir les informations relatives à l'activité de cet organe et connectés à un émetteur porté par le patient et alimenté par une source d'énergie électrique autonome et un récepteur des signaux de l'émetteur, ledit récepteur étant connecté à une entrée d'un convertisseur de courbe vidéo dont une autre entrée est connectée à la sortie desdits moyens de prise de vue, ledit convertisseur de courbe vidéo étant destiné à permettre l'enregistrement et/ou l'observation synchronisés des images relatives aux évolutions du patient prises par les moyens de prise de vue et celles du diagramme en fonction du temps du fonctionnement de l'organe considéré, ledit récepteur étant en outre connecté à une entrée d'un fréquencemètre vidéo dont une autre entrée est connectée à la sortie desdits moyens de prise de vue, ledit fréquencemètre vidéo étant destiné à permettre l'enregistrement et/ou l'observation synchronisés des images relatives aux évolutions du patient prises par les moyens de prise de vue et les images d'informations relatives au fonctionnement de l'image considérée.

Dans une variante, les moyens de conversion des signaux de sortie du récepteur en signaux vidéo comportent une convertisseur analogique-numérique dont l'entrée est connectée au récepteur et dont la sortie est connectée à un circuit de codage vidéo, la sortie de ce dernier étant connectée à l'entrée correspondante d'un circuit mélangeur par l'intermédiaire d'un circuit de décodage. Ce circuit mélangeur fait partie du fréquencemètre vidéo. Une de des entrées est connectée à une sortie d'un circuit séparateur des signaux provenant des moyens de prise de vue; une autre entrée est reliée à des moyens de conversion des signaux de sortie du récepteur en signaux vidéo. Le convertisseur vidéo est connecté à l'entrée d'enregistrement d'un magnétoscope, la sortie lecture de la piste audio de ce dernier étant reliée à l'entrée du circuit de codage vidéo.

Ce dispositif permet l'enregistrement de données biomédicales de manière synchrone grâce à l'utilisation d'un fréquencemètre qui permet ladite synchronisation, éventuellement avec l'aide de la piste audio d'un magnétoscope. Ce dispositif nécessite ainsi des moyens de synchronisation qui alourdissent le traitement des données et rendent la réalisation du dispositif plus compliquée. De plus, la transmission sur la bande audio se fait de manière brute, indépendamment de la bande passante du signal de la bande son des bandes vidéo, ce qui occasionne des pertes d'information.

Le procédé objet de l'invention a pour objectifs de réduire la taille des éléments entrant en jeu et de permettre une transmission synchronisée et sans perte d'information.

l'invention concerne un procédé de traitement des données biomédicales constituées par un signal vidéo provenant d'un système d'imagerie médical synchrone avec un signal bioélectrique acquis par au moins un capteur bioélectrique. Elle est caractérisée en ce que le signal bioélectrique constitue le signal de modulation d'un signal audiofréquence et en ce que le signal ainsi modulé est transmis de manière synchrone avec le signal vidéo.

Préférentiellement, le signal vidéo et le signal audiofréquence sont transférés respectivement sur la piste vidéo et la piste audio d'un support magnétique.

On peut avantageusement utiliser un multiplexage audio-vidéo pour la transmission des données. Dans une autre variante, le signal audiofréquence constitue la modulation en amplitude du signal audio.

L'invention concerne aussi l'interface pour la mise en oeuvre du procédé. Cette interface peut avantageusement contenir un oscillateur local à une fréquence nominale comprise entre 2b et 4 kHz, au moins deux entrées pour le signal bioélectrique et pour le signal vidéo et au moins une sortie. Préférentiellement, la fréquence nominale est environ égale à 3 kHz. L'interface pour le traitement des informations transmises par une bande vidéo fait aussi l'objet de l'invention.

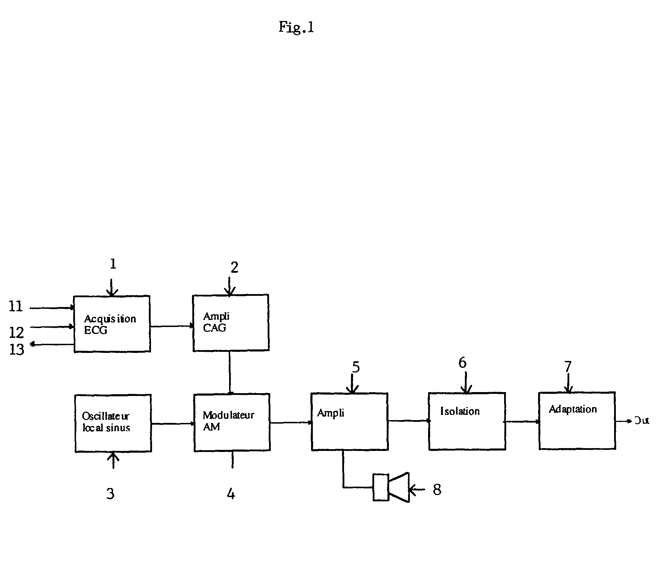

La figure 1 représente le schéma synoptique d'un dispositif selon l'invention.

L'invention sera mieux comprise par la description qui suit d'un exemple de réalisation préférentiel.

Dans cet exemple, le signal obtenu grâce à un électrocardiogramme est couplé avec le signal vidéo d'une échographie. Le signal obtenu grâce à un électrocardiogramme (ECG) est un signal physiologique de faible amplitude qui est recueilli sur le patient par des moyens d'acquisition ECG (1) qui sont ici des électrodes de surface possédant deux entrées et une sortie (11-12-13). La bande passante du signal ECG est de 0,1 Hz à 100 Hz. L'amplitude du signal ECG varie de 0,1 à 2mV et l'impédance de sortie des électrodes est de l'ordre de 5 MΩ. Le signal ainsi recueilli est amplifié par le biais d'un amplificateur à contrôle automatique de gain (CAG) (2) qui égalise le niveau de ces dits signaux et qui peut varier d'un patient à l'autre de manière à avoir un taux constant de modulation.

Un oscillateur local (3) génère un signal sinusoïdal à une fréquence fixe de 3kHz avec, de manière préférentielle, une bonne stabilité en amplitude et en fréquence. La sortie de l'amplificateur CAG et de l'oscillateur local sont connectées à un modulateur AM (4). La sortie de ce modulateur est reliée à l'entrée d'un amplificateur (5) dont la sortie est reliée à l'entrée d'un système d'isolation (6) qui assure la protection du patient par rapport au secteur en isolant complètement l'étage d'acquisition et l'entrée audio du magnétoscope. Des moyens d'adaptation (7) viennent ensuite assurer l'interface avec le magnétoscope. Un haut-parleur (8) peut éventuellement être relié à l'amplificateur pour émettre des signaux d'alerte.

Le signal de sortie de la carte ECG est enregistrable sur la piste audio d'une bande vidéo S-VHS. Ce signal est acheminé sur l'entrée ligne (droite ou gauche) d'un magnétoscope. La sensibilité d'une entrée audio S-VHS est de l'ordre de -5 dB pour une impédance de 50 kQ. Ce signal doit se situer dans la bande passante de la bande son d'une bande vidéo, à savoir 20 Hz à 10000 Hz, pour éviter des pertes d'information.

Afin de protéger le patient de tout risque de choc électrique, le dispositif se présente sous la forme d'un module autonome alimenté par piles rechargeables. L'encombrement de ce dit module doit être réduit pour permettre des manipulations plus faciles. D'autre part, le boîtier isole l'électronique de la carte des perturbations extérieures pour empêcher une propagation de ce signal de faible amplitude à la sortie de haute impédance des électrodes. La carte est ainsi intégrée dans un boîtier étanche avec un compartiment pour les batteries.

## Revendications

1. Procédé de traitement de données biomédicales constituées par au moins un signal vidéo provenant d'un système d'imagerie médical synchrone avec au moins un signal bioélectrique acquis par au moins un capteur bioélectrique caractérisé en ce que le signal bioélectrique constitue le signal de modulation d'un signal audiofréquence et en ce que le signal ainsi modulé est transmis de manière synchrone avec le signal vidéo.

2. Procédé de transmission de données biomédicales selon la revendication principale caractérisé en ce que le signal vidéo et le signal audiofréquence sont transférés respectivement sur la bande vidéo et la bande audio d'un support magnétique.

3. Procédé de transmission de données biomédicales selon l'une quelconque des revendications précédentes caractérisé en ce que l'on utilise un multiplexage audio-vidéo.

4. Procédé de transmission de données biomédicales selon l'une quelconque des revendications précédentes caractérisé en ce que le signal audiofréquence constitue la modulation en amplitude du signal audio.

5. Interface pour un boîtier permettant la mise en oeuvre du procédé de la revendication 1 contenant un oscillateur local à une fréquence nominale comprise entre 2b et 4 kHz, au moins deux entrées pour le signal bioélectrique et pour le signal vidéo et au moins une sortie.

6. Interface pour un boîtier selon la revendication 5 caractérisée en ce la fréquence nominale est environ égale à 3 kHz.
